# EUROPÄISCHE PATENTSCHRIFT

(11) **EP 1 910 276 B1**
(45) Veröffentlichungstag und Bekanntmachung des Hinweises auf die Patenterteilung: **26.05.2010**
(21) Anmeldenummer: 06762415.5
(22) Anmeldetag: 05.07.2006
(51) Int. Cl.: C07C 253/20

(54) **VERFAHREN ZUR HERSTELLUNG VON NITRILEN DURCH ELIMINIERUNGSREAKTIONEN**
PROCESS FOR PREPARING NITRILES BY ELIMINATION REACTIONS
PROCEDE DE PRODUCTION DE NITRILES PAR REACTIONS D'ELIMINATION

(30) Priorität: 25.07.2005 DE 102005035250
(43) Veröffentlichungstag der Anmeldung: 16.04.2008
(73) Patentinhaber: Archimica GmbH, 65929 Frankfurt am Main (DE)
(72) Erfinder: MEUDT, Andreas, 65719 Hofheim (DE); NERDINGER, Sven, 83088 Kiefersfelden (DE); BÖHM, Claudius, 60594 Frankfurt am Main (DE)
(74) Vertreter: Schweitzer, Klaus
(86) Internationale Anmeldenummer: PCT/EP2006/006548
(87) Internationale Veröffentlichungsnummer: WO 2007/012387

(56) Entgegenhaltungen:
- WO-A-99/37611
- WO-A-2005/070879
- DE-A1- 10 063 493
- GLAUDER, J.: "T3P: Propane phosphonic acid anhydride for peptide & amide synthesis" INTERNET CITATION, [Online] März 2004 (2004-03), XP002403967 Gefunden im Internet: URL:http://www.archimica.com/images/Librar y_Files/030004CPHASpecChemT3PTechArticle.p df> [gefunden am 2006-10-20]
- ANONYM: "T3P: THE WATER SCAVENGER. T3P (PROPANE PHOSPHONIC ACID ANHYDRIDE) - A VERSATILE CONDENSATION REAGENT" INTERNET CITATION, [Online] Juni 1999 (1999-06), XP002337773 Gefunden im Internet: URL:http://lse.clariant.com/lse/e2wtools.n sf/lookupDownloads/T3Pnew.pdf/$ FILE/T3Pnew.pdf> [gefunden am 2005-07-25]
- MARCH, J.: "Advanced Organic Chemistry, second edition" ADVANCED ORGANIC CHEMISTRY, MCGRAW-HILL, US, 1977, Seiten 953-954, XP002331027

## Beschreibung

Nitrile sind bedeutsame und äußerst vielseitig verwendbare Zwischenprodukte in der organischen Synthese. Sie zeigen eine hohe Reaktivität der C,N-Mehrfachbindung, wodurch zahllose Heterocarbonyl-Reaktionen ermöglicht werden. Die Bedeutung in der modernen organischen Synthese wird nur durch Limitierungen der Zugänglichkeit dieser Verbindungsklassen eingeschränkt. Standardverfahren zur Herstellung von Nitrilen sind Dehydratisierungen von Carbonsäureamiden, wobei zahllose Reagenzien wie beispielsweise POCl3 eingesetzt werden können.

In der modernen organischen Synthese nimmt die Bedeutung chemo-, regio- und stereoselektiver Reagenzien explosionsartig zu. Will man beispielsweise in einem komplexen Molekül mit zahlreichen, teilweise nur geringe Reaktivitätsunterschiede aufweisenden funktionellen Gruppen eine bestimmte Säuregruppe in ein Amid überführen, ohne dabei andere Gruppen zu beeinflussen (z.B. Epimerisierung chiraler Funktionalitäten), können unselektive Methoden wie SOCl₂ keine Anwendung mehr finden. Ein hochselektives Reagenz zur äußerst selektiven Amidbindungsknüpfung, das selbst in Oligopeptiden ausgezeichnete Selektivitäten und Ausbeuten ohne Epimerisierung erbringt, ist Propanphosphonsäureanhydrid (T3P®). Dieses Reagenz ist in verschiedenen Lösungsmitteln kommerziell erhältlich und bequem einsetzbar. Die Performance dieses Reagenzes ist so hoch, dass das Problem der Knüpfung von Amid- und Peptidbindungen in komplexen Molekülen heute als gelöst betrachtet werden kann.

Eine vergleichbare Problemlösung für die Überführung von Carbonsäuren und N-Alkylcarbonsäureamide in die entsprechenden Nitrile fehlte bisher. Die bekannten Reagenzien können zwar die gewünschten Transformationen bewerkstelligen, dabei werden aber oft andere Gruppierungen ebenfalls beeinflusst. In vielen Fällen werden durch die drastischen erforderlichen Bedingungen selbst entfernt stehende Stereozentren epimerisiert.

Es wäre daher sehr wünschenswert, ein Verfahren zu haben, das Carbonsäuren und N-Alkylcarbonsäureamide durch Dehydratisierung in die entsprechenden Nitrile überführen kann, dabei aber gleichzeitig sehr hohe Chemoselektivitäten aufweist, und zusätzlich in wirtschaftlich nutzbaren Verfahren einsetzbar ist. Die bekannten Reagenzien lösen dieses Problem nicht, wie an einigen Beispielen demonstriert werden soll: POCl3 in Kombination mit Basen kann die genannten Reaktionen zwar bewerkstelligen, allerdings reagiert fast jede mögliche funktionelle Gruppe ebenfalls mit diesem Reagenz. Mit Dicyclohexylcarbodiimid (DCC) kann man ebenfalls die gewünschte Transformation in Nitrile durchführen, allerdings treten hierbei häufig partielle Epimerisierungen auf; schlimmer noch sind häufig aber die Eigenschaften des als Folgeprodukt gebildeten Dicyclohexylharnstoffs, der oft kaum oder nur durch chromatographische Trennungen vom Produkt getrennt werden kann. Die Verwendung von wasserlöslichen DCC-Derivaten ist meist durch deren sehr hohen Preis und schwierige Zugänglichkeit nicht wirtschaftlich durchführbar.

XP002403967 (Glauder, J.: "T3P: Propane phosphonic acid anhydride for peptide & amide synthesis", Internet Citation [Online] (2004-03), found on the Internet: URL http: //www.archimica.com/images/LibraryFiles/030004CPHASpecChem-T3PTechArticle.pdf) beschreibt die Herstellung von Nitrilen durch Reaktion von Carboxamiden oder Carbonsäuren mit Propanphosphonsäure Anhydriden (T3P) in Anwesenheit von Ammoniumchlorid unter milden Bedingungen (45°C).

XPO02337773 ("T3P: The water scavenger. T3P (Propane phosphonic acid anhydride) -A versatile condensation reagent", Internet Citation [Online] (1999-06) found on the Internet: URL:http://lse.clariant.com/Ise/e2wtools.nsf/lookupDownloads-/T 3Pnew.pdf/$FILE/T3Pnew.pdf) beschreibt Propanphosphonsäure Anhydride (T3P) und deren Verwendung als effektives Kondensations-Agens, z.B. in der Synthese von Peptiden, in Veresterungsreaktionen, Epoxidierungen oder Polykondensationsreaktionen.

WO 99/37611 A beschreibt die Herstellung von Amiden durch Reaktion von Ammoniakgas mit einer Carbonsäure in Gegenwart von Propanphosphonsäure Anhydriden.

DE 100 63 493 A1 beschreibt die Herstellung von Ketonen durch Reaktion von Benzoesäure mit CH-aziden Verbindungen in Gegenwart von Propanephosphonsäure Anhydriden.

XP002331027 (March, J., Advanced Organic Chemistry, 2nd edition, MCGRAW-HILL, US (1977), pp. 953-954) beschreibt die Herstellung von Nitrilen aus Amiden mit Hilfe von phosphorhaltigen Dehydratisierungsagenzien wie Phosphorpentoxid oder aus N-alkyl-substituierten Amiden in Gegenwart von PCl₅ (von Braun Reaktion).

Überraschenderweise wurde gefunden, dass Alkylphosphonsäureanhydride alle diese Probleme lösen und ideale und hochselektive Reagenzien zur Überführung von Carbonsäuren und N-Alkylcarbonsäureamide durch Dehydratisierung in die entsprechenden Nitrile sind, wobei gleichzeitig die gewünschte Epimerisierungsfreiheit und maximale Regio- und Stereoselektivität beobachtet wird.

Die vorliegende Erfindung betrifft somit ein hochselektives Verfahren zur Herstellung von Nitrilen der Formel (II),

R - C ≡ N (II)

durch Umsetzung von
- N-Alkylcarbonsäureamiden (RCO-NHR¹) oder
- Ammoniumsalzen von Carbonsäuren (RCOO-NH₃R¹⁺) oder
- Carbonsäuren in Gegenwart von Alkylaminen oder Ammoniumsalzen (RCOOH + NH₂R¹, RCOOH + NH₃R¹⁺)
wobei R für einen beliebig substituierten linearen oder verzweigten C₁-C₁₂-Alkylrest, einen C₃-C₁₂-Cycloalkyl-, Alkenyl-, Alkinyl- oder einen Aryl- oder Heteroarylrest und R¹ für einen beliebigen substituierten, linearen oder verzweigten C₂-C₁₂-Alkylrest, einen C₃-C₁₂-Cycloalkyl-, Alkenyl-, Alkinylrest steht, mit Phosphonsäureanhydriden gegebenenfalls in Gegenwart einer Base in einem organischen Lösungsmittel bei einer Temperatur im Bereich von -30 bis 180°C.

In einer bevorzugten erfindungsgemäßen Ausführungsform ist das Phosphonsäureanhydrid ein 2,4,6-substituiertes 1,3,5,2,4,6-trioxatriphosphinan-2,4,6-trioxid der Formel (I), 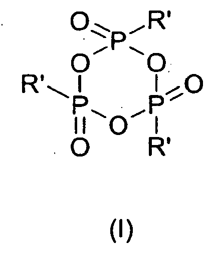 und R' unabhängig voneinander für offenkettige oder verzweigte, gesättigte oder ungesättigte, geradkettige C₁ bis C₁₆-Alkylreste, insbesondere einen C₂ bis C₁₂-Alkylrest, oder cyclische C₃ bis C₁₆-Alkylreste oder für Aryl oder Heteroaryl steht. Besonders bevorzugt werden Phosphonsäureanhydride der Formel (I), in denen R' für einen Methyl, Ethyl, n-Propyl, Isopropyl, n-Butyl, 2-Butyl, Isobutyl, Pentyl, Hexyl, insbesondere einen Ethyl-, Propyl- und/oder Butyl-Rest steht, ganz besonders bevorzugt Propanphosphonsäureanhydrid (T3P).

Die Dehydratisierung zu Nitrilen (II) wird dabei im allgemeinen bei Temperaturen im Bereich von -30 bis +180°C durchgeführt, bevorzugt sind Temperaturen im Bereich von 0 bis 120°C, insbesondere +30 bis +70°C, wobei tiefere Temperaturen im allgemeinen mit höheren Selektivitäten korreliert sind. Die Reaktionsdauer ist abhängig von der angewandten Temperatur und beträgt im allgemeinen 1 bis 12 Stunden, insbesondere 3 bis 6 Stunden.

Das Phosphonsäureanhydrid kann dem Reaktionsmedium entweder als Schmelze oder als flüssige Mischung gelöst in einem Lösungsmittel zugegeben werden. Geeignete Lösungsmittel sind dabei solche, die keine Nebenreaktionen mit dem Phosphonsäureanhydrid ergeben, dies sind alle aprotischen organischen Lösungsmittel, wie z.B. Ligroin, Butan, Pentan, Hexan, Heptan, Octan, Cyclopentan, Cyclohexan, Cycloheptan, Cyclooctan, Dichlormethan, Chloroform, Tetrachlorkohlenstoff, 1,2-Dichlorethan, 1,1,2,2-Tetrachlorethan, Methylacetat, Ethylacetat, Propylacetat, Butylacetat, Dimethylformamid, Diethylformamid, Dimethylacetamid, Diethylacetamid, Diethylether, Diisopropylether, tert-Butyl-Methylether, THF, Dioxan, Acetonitril oder Gemische aus diesen, besonders bevorzugt sind Dichlormethan, Chloroform, Ethylacetat, Propylacetat, Butylacetat, Dimethylformamid, Diethylformamid, Dimethylacetamid, Diethylacetamid, Diisopropylether, tert-Butyl-Methylether, THF, Dioxan, Acetonitril oder Gemische aus diesen, ganz besonders bevorzugt werden Dichlormethan, Chloroform, Ethylacetat, Butylacetat, Dimethylformamid, Dimethylacetamid, tert-Butyl-Methylether, THF, Dioxan, Acetonitril oder Gemische aus diesen, insbesondere bevorzugt sind THF, Ethylacetat oder Butylacetat.

Die Zugabe des Phosphonsäureanhydrids erfolgt mindestens stöchiometrisch in Bezug auf die Ausgangsverbindung (Carbonsäure), kann aber auch überstöchiometrisch sein, beispielsweise im Verhältnis 2:1.

Die Umsetzungen werden bevorzugt so durchgeführt, dass das entsprechende N-Alkylamid in einem Lösungsmittel vorgelegt wird, dann auf die Reaktionstemperatur erwärmt und anschließend durch Zudosieren des Phosphonsäureanhydrids als Schmelze oder Lösung in einem der vorstehend genannten Lösungsmittel dieses in das gewünschte Nitril überführt wird.

Die Isolierung des Reaktionsproduktes erfolgt bevorzugt durch Hydrolyse und einfache Phasentrennung, da die Folgeprodukte der Phosphonsäureanhydride allgemein sehr gut wasserlöslich sind. Je nach Natur des zu isolierenden Produkts können dabei auch Nachextraktionen erforderlich sein. Das gebildete Phosphonsäureanhydrid-Folgeprodukt stört Folgereaktionen oft nicht, so dass auch der direkte Einsatz der erhaltenen Reaktionslösungen oft sehr gute Ergebnisse bringt.

In einer weiteren bevorzugten Ausführungsform wird ein Ammoniumsalz einer Carbonsäure (RCOO-NH₃R¹⁺) durch einfaches Erwärmen mit dem Phosphonsäureanhydrid analog dem oben beschriebenen Verfahren in ein Nitril überführt. Hieraus ergibt sich auch ein sehr elegantes und ebenfalls äußerst selektives Verfahren zur direkten Überführung von Carbonsäuren in Nitrile dergestalt, dass man ein beliebiges Ammoniumsalz zur Carbonsäure gibt und anschließend in Gegenwart einer Base mit einem Phosphonsäureanhydrid umsetzt.

Geeignete Basen sind beispielsweise tertiäre Amine wie Triethylamin, Tripropylamin, Benzyldimethylamin, N,N-Dimethylanilin oder Pyridin.
Die Base wird üblicherweise in einem Verhältnis von 1 bis 5 Äquivalenten, vorzugsweise 1 bis 3, insbesondere 1 bis 2, ganz besonders bevorzugt 1 bis 1,2 Äquivalenten, bezogen auf die Carbonsäure zugegeben.

Das Verfahren kann auch so durchgeführt werden, dass eine Lösung oder Suspension der umzuwandelnden Carbonsäure in Kohlenwasserstoffen oder Estern wie Ethyl- oder Butylacetat mit mindestens einem Äquivalent N-Alkylamin versetzt und nachfolgend mit dem Phosphonsäureanhydrid behandelt wird.

Alle benannten Verfahrensweisen zeichnen sich durch sehr gute Ausbeuten (typisch 90-100 %, insbesondere > 95 %) und einfache Aufarbeitung bei gleichzeitiger Abwesenheit von Nebenreaktionen sowie von Epimerisierungen aus. Die Selektivitäten und die Produktreinheiten der erfindungsgemäßen Reaktion liegen im Bereich von 97-100 %, insbesondere 99-100 %.

Das erfindungsgemäße Verfahren soll durch die nachfolgenden Beispiele erläutert werden:

### Beispiel 1: Benzonitril aus Benzoesäure

1 mol Benzoesäure, 1,1 mol tert-Butylamin und 2 mol NEt₃ werden in 150 mL Ethylacetat vorgelegt. 1,2 mol T3P-Lösung in Ethylacetat (50 % w/w) werden im Laufe einer Stunde zudosiert, anschließend wird weitere drei Stunden bei dieser Temperatur nachgerührt. Zu dieser Zeit zeigte das Reaktions-GC einen Umsatz von 100 % an. Die Reaktionsmischung wird 3 Stunden zum Sieden erhitzt und nach Abkühlen auf Raumtemperatur wurden 180 mL Wasser zugeben und die Phasen getrennt. Die organische Phase wurde destilliert. Die isolierte Ausbeute dieser Reaktion betrug 96 %, HPLC-Reinheit 98 % (a/a).

### Beispiel 2: 4-Methoxybenzonitril aus 4-Methoxybenzoesäure-tert.-octylamid

1 mol 4-Methoxybenzoesäure-tert.-octylamid, und 2 mol NEt₃ werden in 150 mL Ethylacetat vorgelegt. 1,2 mol T3P-Lösung in Ethylacetat (50 % w/w) werden zudosiert. Die Reaktionsmischung wird 3 Stunden zum Sieden erhitzt und nach Abkühlen auf Raumtemperatur wurden 180 mL Wasser zugeben und die Phasen getrennt. Die organische Phase wurde destilliert. Die isolierte Ausbeute dieser Reaktion betrug 96 %, HPLC-Reinheit 98 % (a/a).

### Beispiel 3: Terephthalodinitril aus 4-Cyanobenzoesäure-tert.-octylamid

1 mol 4-Cyanobenzoesäure-tert.-octylamid, und 2 mol NEt₃ werden in 150 mL Ethylacetat vorgelegt. 1,2 mol T3P-Lösu ng in Ethylacetat (50 % w/w) werden zudosiert. Die Reaktionsmischung wird 3 Stunden zum Sieden erhitzt und nach Abkühlen auf Raumtemperatur wurden 180 mL Wasser zugeben und die Phasen getrennt. Die organische Phase wurde destilliert. Die isolierte Ausbeute dieser Reaktion betrug 95 %, HPLC-Reinheit 97 % (a/a).

### Beispiel 4: 4-Brom-3,5-dimethoxybenzonitril aus 4-Brom-3,5-dimethoxy-benzoesäure-tert.-octylamid

1 mol 4-Brom-3,5-dimethoxybenzoesäure-tert.-octylamid, und 2 mol NEt₃ werden in 150 mL Ethylacetat vorgelegt. 1,2 mol T3P-Lösung in Ethylacetat (50 % w/w) werden zudosiert. Die Reaktionsmischung wird 3 Stunden zum Sieden erhitzt und nach Abkühlen auf Raumtemperatur wurden 180 mL Wasser zugeben und die Phasen getrennt. Die organische Phase wurde destilliert. Die isolierte Ausbeute dieser Reaktion betrug 95 %, HPLC-Reinheit 97% (a/a).

## Patentansprüche

1. Verfahren zur Herstellung von Nitrilen der Formel (II)
R-C ≡ N (II)
durch Umsetzung von
N-Alkylcarbonsäureamiden der Formel RCO-NHR¹ oder
Ammoniumsalzen von Carbonsäuren der Formel RCOO-NH₃R¹⁺ oder
Carbonsäuren in Gegenwart von Alkylaminen oder Ammoniumsalzen der Formeln RCOOH + NH₂R¹, RCOOH + NH₃R¹⁺
wobei
R für einen beliebig substituierten linearen oder verzweigten C₁-C₁₂-Alkylrest, einen C₃-C₁₂-Cycloalkyl-, Alkenyl-, Alkinyl- oder einen Aryl- oder Heteroarylrest und
R¹ für einen beliebigen substituierten, linearen oder verzweigten C₂-C₁₂-Alkylrest, einen C₃-C₁₂-Cycloalkyl-, Alkenyl-, Alkinylrest steht,
mit Phosphonsäureanhydriden gegebenenfalls in Gegenwart einer Base in einem organischen Lösungsmittel bei einer Temperatur im Bereich von -30 bis 180°C.

2. Verfahren nach Anspruch 1, **dadurch gekennzeichnet, dass** das Phosphonsäureanhydrid ein 2,4,6-substituiertes 1,3,5,2,4,6-trioxatriphosphinan-2,4,6-trioxid der Formel (I) ist, 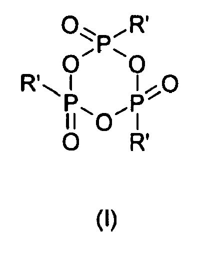 wobei
R' unabhängig voneinander für offenkettige oder verzweigte, gesättigte oder ungesättigte, geradkettige C₁ bis C₁₆-Alkylreste oder cyclische C₃ bis C₁₆₋Alkylreste oder für Aryl oder Heteroaryl steht.

3. Verfahren nach Anspruch 2, **dadurch gekennzeichnet, dass** R' für Methyl, Ethyl, n-Propyl, Isopropyl, n-Butyl, 2-Butyl, Isobutyl, Pentyl oder Hexyl steht.

4. Verfahren nach Anspruch 2, **dadurch gekennzeichnet, dass** das Phosphonsäureanhydrid Propanphosphonsäureanhydrid ist.

5. Verfahren nach einem der vorhergehenden Ansprüche, **dadurch gekennzeichnet, dass** das Phosphonsäureanhydrid entweder als Schmelze oder gelöst in einem Lösungsmittel der Amid- oder Formamid- enthaltenden Reaktionslösung zugegeben wird.

6. Verfahren nach Anspruch 5, **dadurch gekennzeichnet, dass** das Phosphonsäureanhydrid in einem aprotischen Lösungsmittel zugegeben wird.

7. Verfahren nach Anspruch 5, **dadurch gekennzeichnet, dass** das Phosphonsäureanhydrid in einem aprotischen Lösungsmittel in einem Verhältnis von 1:1 bis 2:1, bezogen auf die Carbonsäure, zugegeben wird.

8. Verfahren nach einem der vorhergehenden Ansprüche, **dadurch gekennzeichnet, dass** als Base ein tertiäres Amin der Reaktionsmischung zugesetzt wird.

9. Verfahren nach Anspruch 8, **dadurch gekennzeichnet, dass** Triethylamin, Tripropylamin, Benzyldimethylamin, N,N-Dimethylanilin oder Pyridin als Base zugesetzt wird.

10. Verfahren nach einem der vorhergehenden Ansprüche, **dadurch gekennzeichnet, dass** die Base in einem Verhältnis von 1 bis 2 Äquivalenten, bezogen auf die Carbonsäure, zugegeben wird.

11. Verfahren nach einem der vorhergehenden Ansprüche, **dadurch gekennzeichnet, dass** die Base in einem Verhältnis von 1 bis 1,2 Äquivalenten, bezogen auf die Carbonsäure, zugegeben wird.

12. Verfahren nach mindestens einem der vorhergehenden Ansprüche, **dadurch gekennzeichnet, dass** die Reaktionslösung nach Zugabe des Phosphonsäureanhydrids auf die Reaktionstemperatur erhitzt wird.

## Claims

1. Process for preparing nitriles of the formula (II)
R - C = N (II)
by reaction of
N-alkylcarboxylic acid-amides of the formula RCO-NHR¹ or ammonium salts of carboxylic acids of the formula RCOO-NH₃R¹⁺ or carboxylic acids in presence of alkylamines or ammonium salts of the formulas RCOOH + NH₂R¹, RCOOH + NH₃R¹⁺
wherein
R stands for an arbitrarily substituted linear or branched C₁-C₁₂-alkyl radical, a C₃-C₁₂-cyclo alkyl-, alkenyl-, alkinyl- or an aryl- or heteroaryl radical and
R¹ stands for an arbitrarily substituted linear or branched C₂-C₁₂ alkyl radical, a C₃-C₁₂-cyclo alkyl-, alkenyl-, alkinyl radical,
with phosphonic acid anhydrides if applicable in presence of a base in an organic solvent at a temperature within the range of -30 to 180°C.

2. Method according to claim 1, **characterized in that** the phosphonic acid anhydride is a 2,4,6-substituted 1,3,5,2,4,6-trioxatriphosphinane-2,4,6-trioxide of formula (I), 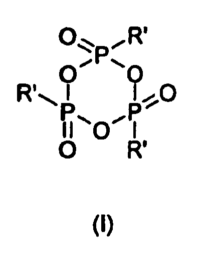 wherein
R' independently from one another stands for open-chained or branched, saturated or unsaturated straight-chained C₁ to C₁₆-alkyl radicals or cyclic C₃ to C₁₆-alkyl radicals or for aryl or heteroaryl.

3. Method according to claim 2, **characterized in that** R' stands for methyl, ethyl, n-propyl, isopropyl, n-butyl, 2-butyl, isobutyl, pentyl or hexyl.

4. Method according to claim 2, **characterized in that** the phosphonic acid anhydride is propane phosphonic acid anhydride.

5. Method according to one of the preceding claims, **characterized in that** the phosphonic acid anhydride is added either as a melt or dissolved in a solvent of the amide- or formamide-containing reaction solution.

6. Method according to claim 5, **charaterized in that** the phosphonic acid anhydride is added in an aprotic solvent.

7. Method according to claim 5, **charaterized in that** the phosphonic acid anhydride is added in an aprotic solvent in a ratio from 1:1 to 2:1 based on the carboxylic acid.

8. Method according to one of the preceding claims, **characterized in that** as a base a tertiary amine is added to the reaction mixture.

9. Method according to claim 8, **characterized in that** triethylamine, tripropylamine, benzyldimethylamine, N,N-dimethylaniline or pyridine is added as base.

10. Method according to one of the preceding claims, **characterized in that** the base is added in a ratio from 1 to 2 equivalents based on the carboxylic acid.

11. Method according to one of the preceding claims, **characterized in that** the base is added in a ratio from 1 to 1.2 equivalents based on the carboxylic acid.

12. Method according to at least one of the preceding claims, **characterized in that** the reaction solution is heated to the reaction temperature after addition of the phosphonic acid anhydride.

## Revendications

1. Procédé de fabrication de nitriles de formule (II) :
R - C = N (II)
en faisant réagir :
des amides d'acides N-alkylcarboxyliques de formule RCO-NHR¹ ou
des sels d'ammonium d'acides carboxyliques de formule RCOO - NH₃R¹⁺ ou
des acides carboxyliques, en présence d'alkylamines ou de sels d'ammonium de formules RCOOH + NH₂R¹, RCOOH + NH₃R¹⁺,
dans lesquelles
R représente un radical alkyle en C₁-C₁₂ linéaire ou ramifié, substitué de manière quelconque, un radical cycloalkyle en C₃-C₁₂, un radical alcényle, un radical alcynyle ou un radical aryle ou hétéroaryle, et
R¹ représente un radical alkyle en C₂-C₁₂ linéaire ou ramifié, substitué de manière quelconque, un radical cycloalkyle en C₃-C₁₂, un radical alcényle, un radical alcynyle,
avec des anhydrides d'acide phosphonique, éventuellement en présence d'une base dans un solvant organique, à une température dans la plage de -30 à 180 °C.

2. Procédé selon la revendication 1, **caractérisé en ce que** l'anhydride d'acide phosphonique est le 1,3,5,2,4,6-trioxatriphosphinan-2,4,6-trioxyde substitué en positions 2,4,6, de formule (I) : 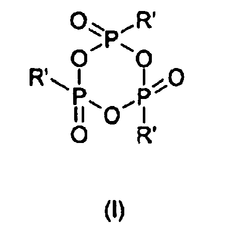 dans laquelle :
les radicaux R' représentent, indépendamment l'un de l'autre, des radicaux alkyle en C₁-C₁₆ à chaîne linéaire, ouverte ou ramifiée, saturée ou insaturée, ou des radicaux alkyle en C₃-C₁₆ cycliques ou un aryle ou un hétéroaryle.

3. Procédé selon la revendication 2, **caractérisé en ce que** R' représente un groupement méthyle, éthyle, n-propyle, isopropyle, n-butyle, 2-butyle, isobutyle, pentyle ou hexyle.

4. Procédé selon la revendication 2, **caractérisé en ce que** l'anhydride d'acide phosphonique est l'anhydride d'acide propanephosphonique.

5. Procédé selon l'une quelconque des revendications précédentes, **caractérisé en ce que** l'anhydride d'acide phosphonique est ajouté sous la forme d'une masse fondue ou sous forme dissoute dans un solvant à la solution réactionnelle contenant un amide ou un formamide.

6. Procédé selon la revendication 5, **caractérisé en ce que** l'anhydride d'acide phosphonique est ajouté à un solvant aprotique.

7. Procédé selon la revendication 5, **caractérisé en ce que** l'anhydride d'acide phosphonique est ajouté à un solvant aprotique dans un rapport de 1:1 à 2:1 par rapport à l'acide carboxylique.

8. Procédé selon l'une quelconque des revendications précédentes, **caractérisé en ce que** l'on ajoute, comme base, une amine tertiaire au mélange réactionnel.

9. Procédé selon la revendication 8, **caractérisé en ce que** l'on ajoute de la triéthylamine, de la tripropylamine, de la benzyldiméthylamine, de la N,N-diméthylaniline ou de la pyridine comme base.

10. Procédé selon l'une quelconque des revendications précédentes, **caractérisé en ce que** la base est ajoutée dans un rapport de 1 à 2 équivalents par rapport à l'acide carboxylique.

11. Procédé selon l'une quelconque des revendications précédentes, **caractérisé en ce que** la base est ajoutée dans un rapport de 1 à 1,2 équivalent par rapport à l'acide carboxylique.

12. Procédé selon au moins l'une quelconque des revendications précédentes, **caractérisé en ce que** l'on chauffe la solution réactionnelle à la température réactionnelle après que l'on a ajouté l'anhydride d'acide phosphonique.
